# FASCICULE DE BREVET EUROPEEN

(11) **EP 1 567 205 B1**
(45) Date de publication et mention de la délivrance du brevet: **02.08.2006**
(21) Numéro de dépôt: 03796175.2
(22) Date de dépôt: 05.12.2003
(51) Int. Cl.: A61L 31/12, A61L 31/04, A61L 31/10

(54) **PROTHESE COMPOSITE**
KOMPOSIT-PROTHESE
COMPOSITE PROSTHESIS

(30) Priorité: 06.12.2002 FR 0215468
(43) Date de publication de la demande: 31.08.2005
(73) Titulaire: Textile Hi Tec, 81270 Labastide Rouairoux (FR)
(72) Inventeur: DOMARD, Alain, F-69006 Lyon (FR); HOUARD, William, F-81270 Labastide Rouairoux (FR); CHAUSSARD, Géraldine, F-69003 Lyon (FR)
(74) Mandataire: Hennion, Jean-Claude
(86) Numéro de dépôt international: PCT/FR2003/003604
(87) Numéro de publication internationale: WO 2004/052423

(56) Documents cités:
- EP-A- 0 477 979
- EP-A- 0 625 056
- FR-A- 2 724 563
- FR-A- 2 766 716
- US-A- 5 622 707
- DATABASE WPI Section Ch, Week 199201 Derwent Publications Ltd., London, GB; Class B04, AN 1992-004447 XP002249992 & JP 03 258723 A (UNITIKA LTD) 19 novembre 1991 (1991-11-19)
- DATABASE WPI Section Ch, Week 199247 Derwent Publications Ltd., London, GB; Class A96, AN 1992-386470 XP002249993 & JP 04 285565 A (UNITIKA LTD) 9 octobre 1992 (1992-10-09)
- CHATELET C ET AL: "Influence of the degree of acetylation on some biological properties of chitosan films" BIOMATERIALS, ELSEVIER SCIENCE PUBLISHERS BV., BARKING, GB, vol. 22, no. 3, février 2001 (2001-02), pages 261-268, XP004221084 ISSN: 0142-9612

## Description

La présente invention concerne une prothèse qui est dite composite pour être constituée d'au moins deux composants distincts. Elle concerne plus particulièrement une prothèse composite utilisable en chirurgie, notamment chirurgie pariétale.

Dans le domaine de la chirurgie pariétale , on a déjà proposé des prothèses composites conçues pour prévenir les adhérences post-chirurgicales. Plus particulièrement de telles prothèses sont constituées d'une part d'un élément de renfort conférant à la prothèse sa résistance mécanique et d'autre part d'un élément de surface destiné à prévenir les adhérences post-chirurgicales et permettant la reconstruction cellulaire. L'élément de renfort est notamment un matériau textile, par exemple un tricot. L'élément de surface peut être un film enduit sur une des faces du matériau textile ou encore une matrice polymère dans laquelle le matériau textile est noyé.

La prothèse composite développée par le demandeur sous la dénomination PARP PU en 1997 était constituée d'un tricot tridimensionnel en polyester sur une face duquel était enduit un film de polyuréthanne. Ni le tricot ni le film de polyuréthanne n'était bio-résorbable.

Dans le document EP.625.056, la prothèse composite comprend une matière polymère dans laquelle est noyé un matériau textile, notamment un tricot, à la fois le matériau textile et la matrice polymère étant dans un matériau bio-résorbable. Plus particulièrement le matériau du tricot est choisi dans le groupe constitué par les acides hyaluronique et alginique ou leurs dérivés ; de plus la matrice polymère est dans une matière choisie dans le groupe constitué par l'acide hyaluronique ou un dérivé de celui-ci, l'acide alginique ou un dérivé de celui-ci et un polysaccharide gélifié.

Le document WO.96/08277 concerne l'utilisation de gel de collagène pour la fabrication d'une membrane transparente et bio-résorbable, permettant la prévention des adhérences post-opératoires. Selon une variante de réalisation, le gel de collagène est coulé dans un récipient puis un élément de renfort ou treillis synthétique , résorbable ou non, est déposé sur le gel. Après séchage à l'air , on obtient une prothèse composite constituée d'une membrane continue de collagène d'environ 200µm d'épaisseur adhérant à la surface du treillis synthétique. Après cinq à douze semaines d'implantation , la membrane collagénique associée au treillis synthétique s'est résorbée, sa résorption s'étant accompagnée de la libération spontanée des éventuelles adhérences qui avaient été observées précédemment et d'une parfaite péritonisation du site implanté. Ainsi selon l'enseignement de ce document WO.96/08277, cette prothèse composite permet de favoriser la régénération du péritoine et la prévention des adhérences post-opératoires.

Selon le document FR.2.766.716, la durée de résorption de cinq semaines et plus n'est pas acceptable. Plus précisément selon ce document il peut être contraire au principe de la prévention des adhérences de maintenir le matériau bio-résorbable, sur lequel repose cet effet barrière anti-adhérent , au-delà des huit à dix jours post-opératoires. De plus la résorption relativement lente de la membrane collagénique s'oppose dans certains cas , selon le document FR.2.766.716, à une recolonisation efficace et précoce du treillis synthétique , celui-ci étant masqué par la membrane collagénique au moment où il doit être intégré, c'est-à-dire dans les deux premières semaines.

Pour améliorer, en particulier quant à son temps de résorption, la prothèse composite selon le document WO.96/08277, le document FR.2.766.716 propose une prothèse composite dans laquelle l'élément de renfort est un tissu qui présente une structure tridimensionnelle notamment ajourée et le film bio-résorbable est constitué par au moins un dérivé de polysaccharide formant un hydrogel insoluble en milieu aqueux, notamment un dérivé de l'acide hyaluronique ou l'un de ses sels. L'épaisseur du film de dérivé de polysaccharide est comprise entre environ 30µm et 100µm.

Ainsi, en sélectionnant le dérivé de polysaccharide et en l'associant à un tissu prothétique présentant une structure tridimensionnelle, il serait possible selon le document FR.2.766.716 d'empêcher la colonisation cellulaire immédiate post-chirurgicale sur la face de la prothèse composite comportant le film de dérivé de polysaccharide et ceci pendant une semaine au maximum, ce qui en chirurgie viscérale suffit notamment pour que le péritoine se reconstitue. De plus cette prothèse faciliterait la colonisation cellulaire immédiate post-chirurgicale sur la face comportant le tissu , ouverte en profondeur , de manière à permettre une intégration rapide et mécaniquement efficace de celle-ci , notamment lorsqu'elle est utilisée en tant que renfort pariétal ou viscéral.

La présente invention a pour but de proposer une nouvelle prothèse composite comprenant un matériau textile de renfort dont une face est associée à un dérivé de polysaccharide formant un hydrogel insoluble aux pH biologiques qui diffère des prothèses composites connues en ce qu'elle présente une très grande souplesse, permettant de s'adapter naturellement à la paroi sur laquelle elle est appliquée , en ce qu'elle constitue un apport nutritionnel amélioré pour la recolonisation cellulaire et en ce qu'elle possède une très bonne tenue mécanique.

De manière caractéristique selon l'invention , l'une des faces du matériau textile est associée à une matrice polymère qui est constituée d'un véritable gel physique de chitosane , formant une masse visco-élastique comportant au moins 95% d'eau et ayant une épaisseur d'au moins 1mm, ledit chitosane ayant un degré d'acétylation au plus de 30%.

Une telle matrice polymère est non seulement à-même de former une barrière prévenant les adhérences post-chirurgicales , mais de plus grâce à la quantité d'eau qu'elle contient , elle constitue en elle-même un apport d'eau complémentaire au liquide biologique nécessaire à la colonisation cellulaire à la surface de la prothèse. Une telle barrière a une durée de bio-résorption qui est fonction du degré d'acétylation et également du lieu d'implantation. Lorsque le degré d'acétylation est proche de 30% , la durée de bio-résorption totale est de l'ordre d'un mois.

De préférence le degré d'acétylation est compris entre O et 15% , ce qui conduit à une durée de bio-résorption comprise entre 2 et 3 mois.

De préférence le chitosane a un poids moléculaire élevé, qui correspond à un haut degré de polymérisation, en sorte que cette matrice polymère présente une bonne tenue mécanique qui permet au praticien de manipuler cette prothèse composite sans précaution particulière, par exemple un poids moléculaire de l'ordre ou supérieur à 200.000 g/mol, de préférence d'au moins 300.000 g/mol et encore plus préférentiellement de l'ordre de 400.000 g/mol.

De préférence pour obtenir un tel degré de polymérisation, le gel physique de chitosane est obtenu à partir d'endosquelettes , notamment de calamars.

Il est à noter que lors de la recolonisation cellulaire , il se produit des sérosités , qui peuvent générer de l'inflammation dans le cas où elles ne peuvent s'écouler librement. La présence d'une prothèse peut constituer un obstacle à l'écoulement naturel des sérosités qui risquent de s'accumuler et former un foyer d'inflammation.

S'agissant de la prothèse composite de l'invention, la matrice polymère, constituée par un vrai gel physique de chitosane , permet la diffusion naturelle desdites sérosités dans la structure tridimensionnelle dudit gel.

Néanmoins , en particulier lorsque l'épaisseur de la matrice polymère est importante , il peut être préférable que celle-ci soit perforée pour compléter la diffusion naturelle au sein de sa structure tridimensionnelle par une diffusion capillaire à travers les conduits de perforation.

Bien sûr du fait du caractère visco-élastique de la matrice polymère, les perforations peuvent avoir tendance à se refermer. Cependant lors de la mise en place de la prothèse composite , celle-ci est amenée à être déformée pour adopter la configuration de la paroi sur laquelle elle est appliquée. Cette déformation se répercute également sur les conduits de perforation , ce qui permet d'obtenir globalement une diffusion capillaire des sérosités.

Dans une variante de réalisation , le matériau textile de renfort est un non-tissé, notamment formé de fils continus de polyester.

Dans une autre variante de réalisation, le matériau textile de renfort est un tricot tridimensionnel.

Le matériau de renfort présente généralement une certaine extensibilité, en particulier lorsqu'il s'agit d'un tricot à structure tridimensionnelle. Du fait du caractère visco-élastique de la matrice polymère formée du gel physique de chitosane , ladite matrice peut subir sans aucun inconvénient cette mise sous extension qui peut se produire lors de la manipulation de la prothèse composite par le praticien.

Le matériau textile de renfort peut être éventuellement bio-résorbable, grâce à la mise en oeuvre de fibres ayant cette propriété.

La présente invention sera mieux comprise à la lecture de la description qui va être faite d'une prothèse composite comprenant un non-tissé en polyester comme matériau textile de renfort et une matrice polymère constituée d'un vrai gel physique de chitosane dont le degré d'acétylation est compris entre O et 15%, ladite matrice ayant une épaisseur comprise entre 1 et 3mm.

### EXEMPLE DE PREPARATION D'UNE SOLUTION DE CHITOSANE

On part du chitosane d'endosquelette de calmar dont le poids moléculaire est de l'ordre de 400.000 g/mol, que l'on purifie par les opérations successives suivantes : solubilisation dans l'eau permutée avec un léger excès d'acide acétique (solution à 0,5% (w/v)), filtration sur différentes porosités (3/1,2/0,8 et 0,45µm), précipitation (ajout de quelques gouttes d'une solution ammoniacale 16,5%), lavages dans l'eau permutée jusqu'à neutralité , et séchage par lyophilisation.

Dans un premier temps, le chitosane est solubilisé dans l'eau permutée avec une quantité stoechiométrique d'acide acétique. Après dissolution , un volume de diol équivalent au volume d'eau initial est ajouté : formation d'une solution hydroalcoolique. Le tout est placé sous agitation vive, puis subit un dégazage. La solution est ensuite coulée dans un moule adapté, et laissée au repos tel quel pendant 8 heures. Le traitement thermique de cette solution accélère la gélification et conduit à la formation d'un gel après une durée fonction de la concentration en chitosane , du matériel utilisé , et des conditions de traitement (température et pression). Le gel est ensuite neutralisé dans une solution ammoniacale à 16,5%, et lavé dans l'eau permutée jusqu'à neutralité du milieu. Pour garantir sa stabilité dans le temps , le gel doit être maintenu immergé dans de l'eau permutée ou une solution tampon.

### EXEMPLE DE PREPARATION D'UNE PROTHESE COMPOSITE

On dispose d'un moule ayant la configuration souhaitée pour la prothèse composite , par exemple ayant une configuration carrée de 15 cm de côté. On verse dans ce moule une solution de chitosane dont le degré d'acétylation est compris entre O et 15 % et dont le poids moléculaire est de l'ordre de 400.000g/mol.

On place le non-tissé , formant le matériau textile de renfort , sur la solution de chitosane juste avant le point de gel de celle-ci et on laisse se réaliser la prise en gel.

Il importe de contrôler , par d'éventuels essais préalables, la cinétique de prise en gel de la solution de chitosane , cinétique qui est variable en fonction du degré d'acétylation et du poids moléculaire du matériel utilisé , des conditions de traitement (température et pression), de manière à ce que le matériau textile de renfort qui est déposé sur la surface de la solution ne soit pas totalement traversé par ladite solution. Au contraire il est souhaitable que seuls les fils ou fibres superficielles du matériau textile de renfort soient pénétrés par la solution lors de la prise du gel. Par exemple , dans les conditions visées ci-dessus , le non-tissé a été posé sur la solution gélifiante au bout de 7h3O pour une solution à 1% de chitosane , de 7h pour une solution à 1,5% de chitosane et 6h3O pour une solution à 2% de chitosane, les solutions étant placées dans une étuve à 45°C.

La prothèse composite est ensuite démoulée , neutralisée dans une solution ammoniacale à 16,5% et lavée dans l'eau permutée jusqu'à neutralité du milieu. Pour garantir la stabilité du gel dans le temps, la prothèse composite est maintenue, avant son utilisation, immergée dans de l'eau permutée ou une solution tampon.

## Revendications

1. Prothèse composite comprenant un matériau textile de renfort dont une face est associée à un dérivé de polysaccharide formant un hydrogel insoluble aux pH biologiques **caractérisée en ce que** l'une des faces du matériau textile est associée à une matrice polymère qui est constituée d'un véritable gel physique de chitosane, formant une masse visco-élastique comportant au moins 95% d'eau et ayant une épaisseur d'au moins 1mm et **en ce que** ledit chitosane a un degré d'acétylation au plus de 30%.

2. Prothèse composite selon la revendication 1 **caractérisée en ce que** le chitosane a un poids moléculaire de l'ordre ou supérieur à 200.000 g/mol.

3. Prothèse composite selon la revendication 2 **caractérisée en ce que** le chitosane a un poids moléculaire d'au moins 300.000 g/mol , de préférence 400.000 g/mol.

4. Prothèse composite selon l'une des revendications 1 à 3 **caractérisée en ce que** le degré d'acétylation du chitosane est compris entre 0 et 15 %.

5. Prothèse composite selon l'une des revendications 1 à 4 **caractérisée en ce que** le gel physique de chitosane est obtenu à partir d'endosquelettes notamment de calamars.

6. Prothèse composite selon l'une des revendications 1 à 5 **caractérisée en ce qu'**au moins la matrice polymère comporte des conduits de perforations , aptes à permettre la diffusion capillaire des sérosités.

7. Prothèse composite selon l'une des revendications 1 à 5 **caractérisée en ce que** le matériau textile de renfort est un non-tissé , notamment de fils continus de polyester.

8. Prothèse composite selon l'une des revendications 1 à 5 **caractérisée en ce que** le matériau textile de renfort est un tricot tridimensionnel.

9. Prothèse composite selon l'une des revendications 1à 8 **caractérisée en ce que** , avant son utilisation, elle est immergée dans de l'eau permutée ou une solution tampon.

## Claims

1. A composite prosthesis comprising a textile strengthening material, one face of which is associated with a polysaccharide derivative forming an hydrogel insoluble at biological pHs **characterized in that** one of the faces of the textile material is associated with a polymer matrix which consists of a real physical chitosan gel, forming a viscoelastic mass including at least 95% water and having a thickness of at least 1 mm and **in that** said chitosan has an acetylation level of 30% at the most.

2. The composite prosthesis according to claim 1, **characterized in that** the chitosan has a molecular weight of the order of or larger than 200,000 g/mol.

3. The composite prosthesis according to claim 2, **characterized in that** the chitosan has a molecular weight of at least 300,000 g/mol, preferably 400,000 g/mol.

4. The composite prosthesis according to any of claims 1 to 3, **characterized in that** the acetylation level of the chitosan is between 0 and 15%.

5. The composite prosthesis according to any of claims 1 to 4, **characterized in that** the physical chitosan gel is obtained from endoskeletons notably from squids.

6. The composite prosthesis according to any of claims 1 to 5, **characterized in that** at least the polymer matrix includes conduits of perforations, capable of providing capillary diffusion of serosities.

7. The composite prosthesis according to any of claims 1 to 5, **characterized in that** the textile strengthening material is a non-woven material, notably of continuous polyester yarns.

8. The composite prosthesis according to any of claims 1 to 5, **characterized in that** the textile strengthening material is a three-dimensional knit.

9. The composite prosthesis according to any of claims 1 to 8, **characterized in that**, before its use, it is immersed in permutated water or a buffer solution.

## Patentansprüche

1. Verbundprothese umfassend ein textiles Verstärkungsmaterial, dessen eine Seite mit einem Polysaccharidsderivat, das ein bei biologischen pH unlösbares Hydrogel bildet, verbunden ist, **dadurch gekennzeichnet, daß** eine der Seiten des textilen Materials mit einer Polymermatrix verbunden ist, die aus einem echten physikalischen Chitosangel besteht, das eine viskoelastische Masse bildet, die mindestens 95% Wasser umfaßt und die eine Dicke von mindestens 1 mm aufweist und, dass das genannte Chitosan einen Acetylierungsgrad von höchstens 30% aufweist.

2. Verbundprothese nach Anspruch 1, **dadurch gekennzeichnet, daß** das Chitosan ein Molekulargewicht hat, das in der Großenordnung von 200.000 g/mol oder mehr liegt.

3. Verbundprothese nach Anspruch 2, **dadurch gekennzeichnet, daß** das Chitosan ein Molekulargewicht von mindestens 300.000 g/mol, vorzugsweise 400.000 g/mol hat.

4. Verbundprothese nach einem der Ansprüche 1 bis 3, **dadurch gekennzeichnet, daß** der Acetylierungsgrad des Chitosans zwischen 0 und 15% liegt.

5. Verbundprothese nach einem der Ansprüche 1 bis 4, **dadurch gekennzeichnet, daß** das physikalische Chitosangel aus Endoskeletten, insbesondere von Kalamaren, erhalten ist.

6. Verbundprothese nach einem der Ansprüche 1 bis 5, **dadurch gekennzeichnet, daß** mindestens die Polymermatrix Perforationsleitungen umfaßt, die geeignet sind, die kapillare Diffusion der serösen Flüssigkeiten zu ermöglichen.

7. Verbundprothese nach einem der Ansprüche 1 bis 5, **dadurch gekennzeichnet, daß** das textile Verstärkungsmaterial filzartig ist, insbesondere aus Polyester-Filamentgarn.

8. Verbundprothese nach einem der Ansprüche 1 bis 5, **dadurch gekennzeichnet, daß** das textile Verstärkungsmaterial ein dreidimensionaler Strickstoff ist.

9. Verbundprothese nach einem der Ansprüche 1 bis 8, **dadurch gekennzeichnet, daß** sie vor ihrer Benutzung in permutiertes Wasser oder in eine Pufferlösung eingetaucht ist.
